Europäisches Patentamt

European Patent Office

Office européen des brevets

(11) Publication number: **0018183**

A1

(12) # EUROPEAN PATENT APPLICATION

(21) Application number: 80301171.7

(22) Date of filing: 11.04.80

(51) Int. Cl.³: **C 07 C 9/04,** C 07 C 7/13, C 07 C 7/12

(30) Priority: 18.04.79 GB 7913483

(43) Date of publication of application: 29.10.80 Bulletin 80/22

(84) Designated Contracting States: DE FR NL

(71) Applicant: **CJB DEVELOPMENTS LIMITED, 20, Eastbourne Terrace, London W2 6LE (GB)**

(72) Inventor: **Leppard, Colin James, 21 Eastfield Close Southbourne, Emsworth, Hampshire (GB)**
Inventor: **Shah, Maroof Ali, Dr., Halesby Woolbeggars Lane, Horsell Woking Surrey (GB)**

(74) Representative: **Allard, Susan Joyce et al, BOULT, WADE & TENNANT 27 Furnival street, London EC4A 1PQ (GB)**

(54) Process for the purification of natural gas.

(57) A process for the purification of natural gas to render it suitable for liquefaction comprises passing a stream of natural gas containing water, methanol and carbon dioxide is passed at elevated pressure along line 20 to a vessel A containing a first bed portion 21 of an absorbent material on which water and methanol are absorbed and a second bed portion 22 of an absorbent material on which carbon dioxide is absorbed. A stream of purified natural gas 23 issues from the vessel A and is passed to a liquifaction plant or any other subsequent process or use. A portion of the purified natural gas about 10%, is passed along line 24 to a vessel B which contains a first bed portion 26 and a second bed portion 25 which have previously absorbed water and methanol, and carbon dioxide, respectively. The gas is passed to vessel B at a reduced pressure and the beds of absorbent material thereby regenerated.

-1-

# PROCESS FOR THE PURIFICATION OF NATURAL GAS

The present invention relates to a process for the purification of natural gas.

Natural gas, as distributed from the stations at which it is received from the wells, contains small concentrations of carbon dioxide and of water and methanol vapours, the methanol having been added to the natural gas to prevent hydrate formation.

Natural gas can be conveniently stored as a liquid but, prior to liquefaction, the concentrations of carbon dioxide, water and methanol must be reduced sufficiently to avoid the formation of unwanted solid deposits on cooling. Such deposits would accumulate on the heat exchanger surfaces in the liquefaction plant thereby rendering the heat exchangers less effective than normal and also causing blockages.

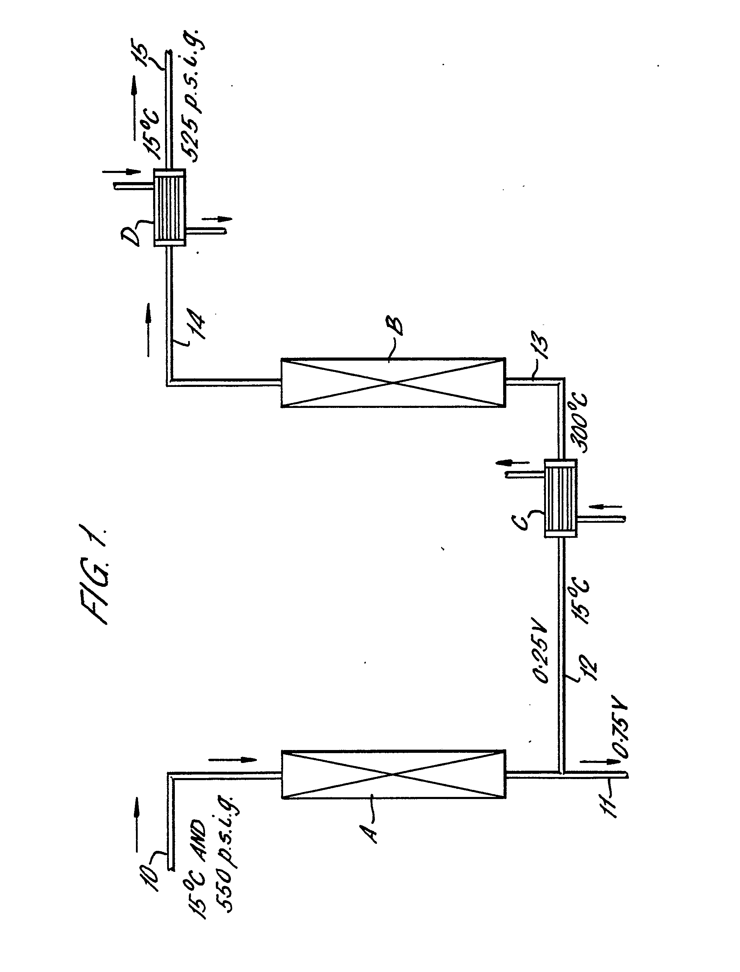

A known purification procedure is illustrated with reference to Figure 1 of the accompanying drawings.

Natural gas at ambient temperature, about 15°C, and at a pressure of about 550 psig is passed via pipe 10 to a bed of a molecular sieve material on which carbon dioxide, water and methanol are absorbed. The approximate impurity level of the natural gas entering the bed A is:

| | |
|---|---|
| Carbon dioxide | upto 20,000 v.p.m. |
| Water | upto saturation value |
| Methanol | 50 v.p.m. |

The purified natural gas then proceeds via pipe 11 to a liquefaction plant.

Saturation of the bed of molecular sieve with the impurities is observed by monitoring the gas outlet and detecting the appearance of carbon dioxide in it. A fresh bed of absorbent material is brought on stream and the saturated bed is regenerated. Regeneration is usually effected by passing a hot stream of previously purified natural gas through the bed at a temperature of about $300^{\circ}C$, this temperature being required to ensure the desorption and removal of water and methanol. To achieve this regeneration, a part, for example, a quarter, of the natural gas emerging from bed A is passed through a heat exchanger C to raise the temperature of the gas to the desired $300^{\circ}C$. The heated natural gas is then passed via pipe 13 through molecular sieve bed B and effects the regeneration of the molecular sieve material. The natural gas emerging from bed B is passed via pipe 14 to a heat exchanger D where it is cooled to $15^{\circ}C$, and then is passed at a pressure of 525 psig. via pipe 15 to a local transmission main.

0018183

One serious disadvantage of this method is that the repeated exposure of the beds of molecular sieve material to alternate heating to about 300°C and cooling is accompanied by a fall in its absorptive capacity per cycle.

Another purification procedure is described in British Patent Specification No. 1419380. This method comprises absorbing water and methanol from a stream of natural gas containing water, methanol and carbon dioxide in a first bed of an absorbent material, and subsequently absorbing the carbon diodixe in a second bed of absorbent material, such as molecular sieve material, regenerating the absorbent material of the said first bed by passing a regenerative gas therethrough at an elevated temperature, preferably from 250° to 350°C, regenerating the absorbent material of the said second bed at a temperature not exceeding 100°C, and then again purifying natural gas to remove therefrom water, methanol and carbon dioxide employing the regenerated materials.

This method also suffers from the disadvantages that the first bed of absorbent material has to be heated to 250° to 350° in order to desorb water and methanol therefrom, with a consequent loss of the absorptive capacity of the absorbent material.

We have now developed a process for the purification of natural gas prior to liquefaction in which alternate heating and cooling of the absorbent material is avoided.

Accordingly, the present invention provides a process for the purification of natural gas (as hereinafter defined) to render it suitable for liquefaction, which process comprises passing a stream of natural gas containing water, methanol and carbon dioxide at elevated pressure, through a first bed of an absorbent material in order to absorb water and methanol therefrom, passing the stream of natural gas after passage through the first bed of absorbent material at elevated pressure through a second bed of absorbent material in order to absorb carbon dioxide therefrom, regenerating the first and second beds of absorbent material by passage of a regenerative gas therethrough at a reduced pressure and thereafter again purifying natural gas to remove therefrom water, methanol and carbon dioxide using the regenerated absorbent materials.

By the term "natural gas" as used herein is meant both natural gas obtained from natural sources and a synthetically produced gas having a composition similar to that of natural gas obtained from natural sources.

Throughout the present specification the terms "absorb" and "absorbent" will be used, but it is to be understood that these terms are intended to include within their meaning the terms "adsorb" and "adsorbent"

0018183

where, in the context, these latter terms would be more appropriate.

The first bed of absorbent material, which absorbs water and methanol from the natural gas stream, may be, for example, silica gel, 3A molecular sieve material activated alumina or any other suitable desiccant.

The second bed of absorbent material, which absorbs carbon dioxide from the natural gas stream, may be, for example, 4A, 5A, 10A or 13X molecular sieve material, activated carbon, activated charcoal or any other suitable absorbent. A mixture of absorbent materials may also be used.

The first and second beds of absorbent materials may be static beds or fluidised beds. The first and second beds of absorbent material may be separate beds which are connected by the necessary pipework. However, in certain instances it may be convenient to form the first and second beds into an integrated bed.

If it is desired, the process of the present invention may be operated continuously and, in order that this may be achieved, it is necessary to provide a second set of first and second absorbent beds which are switched into contact with the natural gas stream when breakthrough of impurities occurs from the absorbing beds of the first set.

-6-

0018183

The first set of absorption beds is then regenerated ready for re-use when breakthrough of impurities occurs from the second set of beds.

The regenerative gas may be any suitable gas, but purified gas is preferred for use as the regenerative gas because of its ready availability and because its use eliminates the need to purge the vessels when they are changed from operating in the absorbing mode to operating in the desorbing mode.

The natural gas is passed through the first and second beds of absorbent material at an elevated pressure, for example about 550 psig, during the absorption of the impurities therefrom. During the regeneration of the beds of absorbent material the regenerative gas, for example purified natural gas, is passed through the first and second beds at a reduced pressure, for example a pressure of about 10 psig.

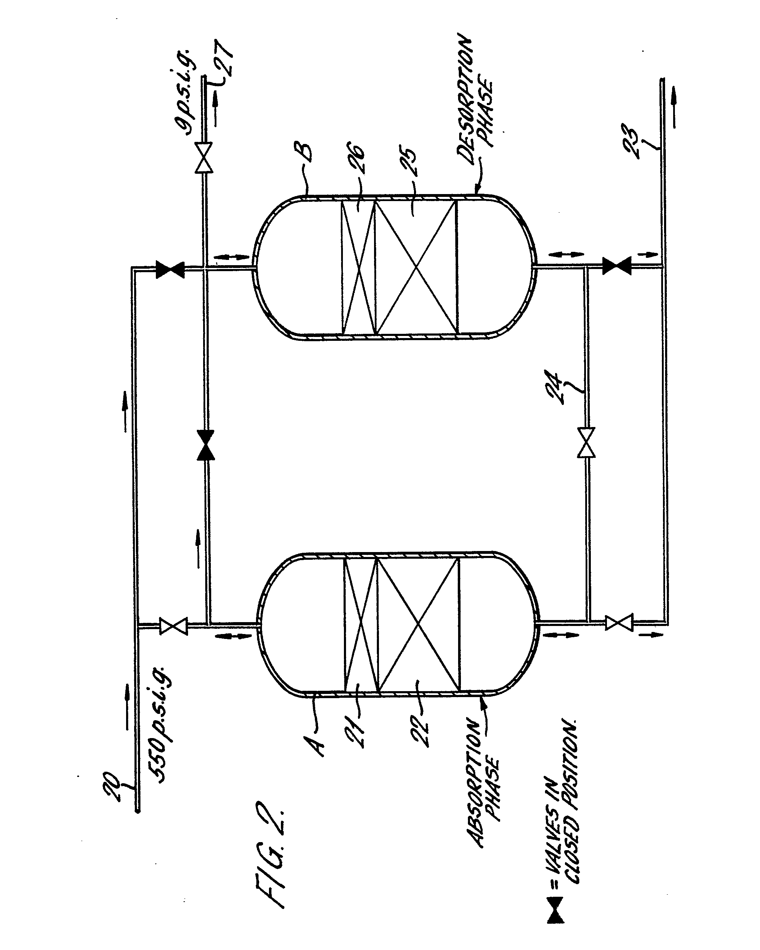

The process of the present invention will be further described with reference to Figure 2 of the accompanying drawings.

Natural gas at a pressure of 550 psig and at ambient temperature, about 15$^{o}$C, is passed via line 20 through a vessel A which contains an integrated bed of absorbent material, comprising a first bed portion 21 of 3000 lbs of silica gel and a second bed portion 22 of 24160 lbs of molecular sieve material. The approximate impurity

0018183

level of the natural gas to be treated is:-

Carbon dioxide       up to 20,000 v.p.m.

water                up to saturation value

methanol             50 v.p.m.

The smaller bed portion 21 in vessel A is intended for the absorption of water and methanol, whilst the larger bed portion 22 is intended for the absorption of carbon dioxide. The proportion of the absorbent materials represented by the bed portion 21 will depend upon the relative proportions of water, methanol and carbon dioxide in the gas to be treated and it will be understood that the optimum choice of relative bed sizes will be readily determinable by one skilled in the art.

The purified natural gas passes along line 23 to a gas liquefaction plant.

A proportion of the purified natural gas, say 10%, issuing from vessel A is passed via pipe 24 to a second vessel B. Vessel B contains an integrated bed of absorbent material comprising a first bed portion 26 of 3000 lbs of silica gel,which has previously absorbed water and methanol and a second bed portion 25 of 24160 lbs of molecular sieve material which has previously absorbed carbon dioxide. The gas stream 24 is passed at a reduced pressure of about 10 psig through bed B. The reduction of pressure of the gas is carried out using apparatus and techniques which are known per se. During passage of the gas stream 24 at a reduced pressure through bed B the carbon dioxide previously absorbed by bed portion

0018183

and the water and methanol previously absorbed by bed portion 26 are desorbed therefrom. The natural gas containing the impurities then passes along line 27 and is then passed to a local main or flared.

It will be understood that each of the vessels A and B will alternate in its function. Thus when vessel A becomes saturated, or almost saturated, the stream of natural gas may be switched by opening and closing appropriate valves to vessel B and during the absorption in vessel B of impurities in the natural gas vessel A undergoes regeneration.

With a feed of natural gas containing the above levels of impurities of 480,000 scfh along line 20 to vessel A an appropriate cycle time is two hours, with approximately 46,000 scfh of the purified gas being passed to vessel B.

It will be understood that the invention also includes within its scope apparatus when used for purifying natural gas in accordance with the method described above, such apparatus being generally of the type as described with reference to Figure 2 of the accompanying drawings.

It will also be understood that whilst an operating pressure of 550 psig is used as an example, there is no specific operating pressure limitation on the practicability of the process.

0018183

CLAIMS

1. A process for the purification of natural gas (as hereinbefore defined) to render it suitable for liquefaction, which process comprises passing a stream of natural gas containing water, methanol and carbon dioxide at elevated pressure through a first bed of an absorbent material in order to absorb water and methanol therefrom, passing the stream of natural gas after passage through the first bed of absorbent material at elevated pressure through a second bed of absorbent material in order to absorb carbon dioxide therefrom, regenerating the first and second beds of absorbent material by passage of a regenerative gas therethrough at a reduced pressure and thereafter again purifying natural gas to remove therefrom water, methanol and carbon dioxide using the regenerated absorbent material.

2. A process as claimed in claim 1 wherein the first bed of absorbent material is a molecular sieve material.

3. A process as claimed in claim 1 wherein the first bed of absorbent material is silica gel.

4. A process as claimed in any one of the preceding claims wherein the second bed of absorbent material is a molecular sieve material.

0018183

5. A process as claimed in any one of the preceding claims wherein natural gas is passed at a pressure of about 550 psig through the first and second beds of absorbent material during the absorption of impurities therefrom, and the first and second beds are regenerated by passage of a regenerative gas therethrough at a pressure of about 10 psig.

6. A process as claimed in any one of the preceding claims wherein the regenerative gas is natural gas.

7. A process as claimed in any one of the preceding claims wherein the first and second beds of absorbent material form an integrated bed.

8. A process as claimed in any one of claims 1 to 6 wherein the first and second beds of absorbent material are static beds.

9. A process as claimed in any one of claims 1 to 6 wherein the first and second beds of absorbent material are fluidized beds.

10. A process as claimed in any one of the preceding claims which is operated continuously by using two sets of first and second absorbent beds, one set of first and second absorbent beds effecting absorption of the water, methanol and carbon dioxide from the natural gas stream whilst the second set of first and second absorbent beds is being regenerated, the second set of first and second absorbent beds being switched into contact with the natural gas stream to effect absorption of the water, methanol, and carbon dioxide when or before the beds of the first set are saturated and the beds of the first set being regenerated simultaneously.

SJA/EA 176

# FIG. 1.

10

15°C AND
550 p.s.i.g.

A

D

15°C     15

525 p.s.i.g.

14

B

C

0·25V

12     15°C     300°C     13

11     0·75V

FIG. 2.

A

21

22

ABSORPTION PHASE

550 p.s.i.g.

20

9 p.s.i.g.

27

B

26

25

DESORPTION PHASE

24

23

▶◀ = VALVES IN CLOSED POSITION.

2/2

| Category | DOCUMENTS CONSIDERED TO BE RELEVANT | | CLASSIFICATION OF THE APPLICATION (Int. Cl. 3) |
|---|---|---|---|
| | Citation of document with indication, where appropriate, of relevant passages | Relevant to claim | |
| D | GB - A - 1 419 380 (BRITISH GAS CORP.))<br>* Pages 2,4 * | 1-6, 10 | C 07 C  9/04<br>7/13<br>7/12 |
| | DE - B - 1 296 723 (MOBIL OIL CORP.)<br>* Claims * | 1,3 | |
| | HYDROCARBON PROCESSING, vol. 54, April 1975, no.4, Houston, USA "Adsorption drying", page 80. | 1-3, 10 | **TECHNICAL FIELDS SEARCHED (Int.Cl. 3)**<br><br>C 07 C  9/04<br>7/13<br>7/12<br>7/00 |
| | HYDROCARBYN PROCESSING, vol. 54, April 1975, no.4, Houston, USA "Molecular Sieve", page 91. | 1-2, 10 | |
| | FR - A - 1 534 172 (W.R. GRACE & CO.)<br>* Abstract * | 1-2, 9-10 | **CATEGORY OF CITED DOCUMENTS**<br><br>X: particularly relevant<br>A: technological background<br>O: non-written disclosure<br>P: intermediate document |
| | CHEMICAL ABSTRACTS, vol. 80, no.8, February 25, 1974, page 117, no. 38970f. Columbus, Ohio, USA LAKEEV V.P., "Zeolite NaA as a drying agent for natural gas containing methanol vapor". & Gazov. Prom. 1973, (10), 39-42. * Abstract * ./. | 1-2, 6 | T: theory or principle underlying the invention<br>E: conflicting application<br>D: document cited in the application<br>L: citation for other reasons<br><br>&: member of the same patent family, corresponding document |

The present search report has been drawn up for all claims

| Place of search | Date of completion of the search | Examiner |
|---|---|---|
| The Hague | 27-06-1980 | VAN GEYT |

EPO Form 1503.1  06.78

| | DOCUMENTS CONSIDERED TO BE RELEVANT | | CLASSIFICATION OF THE APPLICATION (Int. Cl. 3) |
|---|---|---|---|
| Category | Citation of document with indication, where appropriate, of relevant passages | Relevant to claim | |
| | US - A - 3 751 878 (COLLINS)<br>* Column 2, line 56; claims * | 1,4 | |
| | ---- | | |
| | | | TECHNICAL FIELDS SEARCHED (Int. Cl. 3) |